# EUROPEAN PATENT APPLICATION

(11) **EP 2 351 625 A1**
(43) Date of publication of application: **03.08.2011**
(21) Application number: 09817716.5
(22) Date of filing: 28.09.2009
(51) Int. Cl.: B21G 1/02, A61B 17/06, B23K 26/02, B23K 26/03, B23K 26/08, B23K 26/10, B23K 26/38

(54) **DEVICE FOR FORMING BORE AND METHOD FOR FORMING BORE BY EYELESS SEWING NEEDLE**

(30) Priority: 30.09.2008 JP 2008253614
(71) Applicant: Mani, Inc., Tochigi 321-3231 (JP)
(72) Inventor: KATOH, Kazuaki, Utsunomiya-shi Tochigi 321-3231 (JP); MATSUTANI, Kanji, Utsunomiya-shi Tochigi 321-3231 (JP)
(74) Representative: Barth, Stephan Manuel
(86) International application number: PCT/JP2009/066722
(87) International publication number: WO 2010/038683

(57) **Abstract**

There is provided a hole opening device and a hold opening method for an eyeless needle capable of accurately aligning the center of a needle material and the optical axis of a laser beam as well as opening a hole precisely even with a thin needle material.

A plurality of needle materials 1 are arranged in a row and mounted on a table 21, base ends of several needle materials 1 are photographed by a camera 25, a needle material 1 to be processed is located from the photographed image, and the center location of the needle material is calculated. The optical axis of a laser of a laser oscillator and center location of a base end of the needle material 1 to be processed are matched, and a hole 1c of a predetermined depth is opened by the laser. The plurality of needle materials are shifted, a needle material 1 to be processed next of the plurality of needle materials is located from the image photographed by the camera, the center location of the base end is located from the image of the base end of the needle material, and the plurality of needle materials are shifted so as to match the center location of the base end to be processed and the optical axis of the laser of the laser oscillator.

## Description

### [Technical Field]

The present invention relates to a device for opening a hole in an eyeless needle or surgical suture needle using a laser beam and a hole drilling method for the same. It particularly relates to technology suitable for a thin eyeless needle.

### [Background Art]

With an eyeless needle used in surgical operations, a hole is formed along the length of the needle from a base end and a suture thread is inserted in the hole and fixed by caulking. As a hole opening method, a method using a laser beam has been well known (e.g., Patent Document 1).

However, since the aforementioned hole opening operation is performed by hand, efficiency is not improved and cost is high.

Patent Document 2 has been proposed to resolve this problem. This conventional example will be described next while referring to drawings.

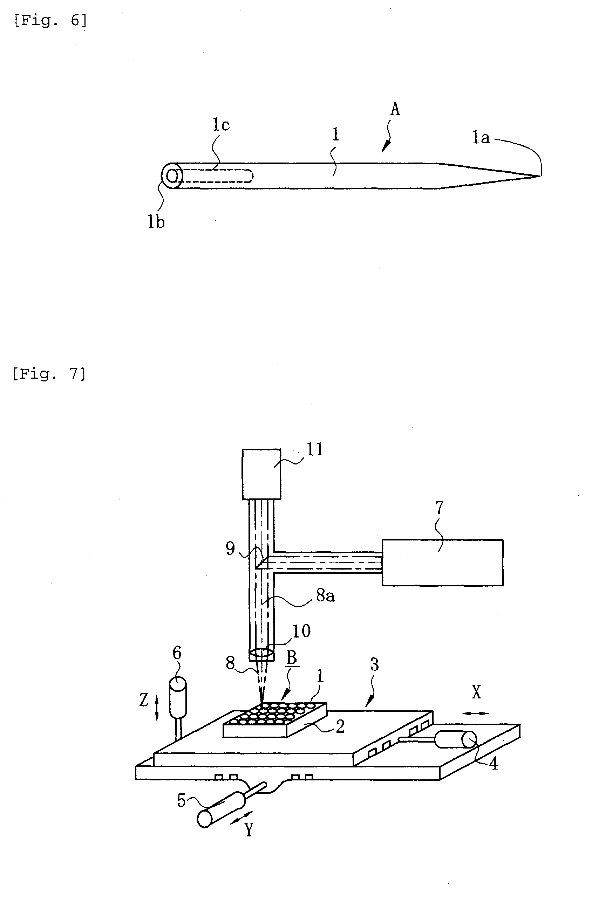

FIG. 6 is an oblique view of a straight eyeless needle before it is curved. This eyeless needle A is a surgical suture needle, and form, diameter, and length thereof differ according to use such as surgery, ophthalmology, and the like. As shown in the drawing, a needle material 1 of this eyeless needle A has a sharp tip 1a, and a base end 1b has a hole 1c opened in the axial direction. A suture thread not illustrated in the drawing is inserted in this hole 1c, and fixed by caulking, and then curved in a predetermined manner. While the illustrated eyeless needle has a circular cross-sectional shape, it may be formed in a triangular, trapezoidal, or flat shape according to purpose.

FIG. 7 is a diagram illustrating the configuration of a conventional hole opening device. Multiple pieces of a needle material 1 before holes are formed are each stored in a container 2 with the base end 1b up and then placed on the table 3.

The table 3 is movable independently in three mutually-orthogonal axial directions by an X-axis drive motor 4, a Y-axis drive motor 5, and a Z-axis drive motor 6. The X-axis drive motor 4, the Y-axis drive motor 5, and the Z-axis drive motor 6 move the table 3 using something capable of moving with high precision, such as ball screws or the like.

A laser oscillator 7 is a device for oscillating a laser beam 8 for opening a hole in the respective base ends 1b of the needle materials 1. The laser beam 8 oscillated by the laser oscillator 7 opens the hole 1c by proceeding downward in the drawing guided by a dichroic mirror 9 and converged by a lens 10 so as for it to be applied to the base end 1b of the needle material 1.

A collection B of the needle materials 1 is photographed by a camera 11, which uses a photoelectric transducer such as a CCD and is provided behind the dichroic mirror 9.

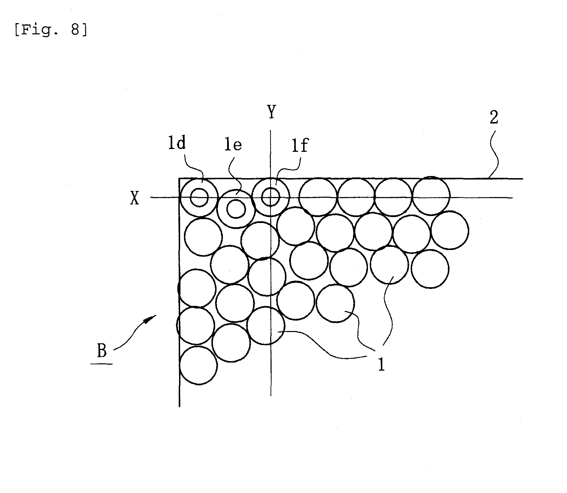

FIG. 8 illustrates a part of an image of the collection B of the needle materials 1 photographed by the camera 11. The center location of each of the needle materials 1 is found by an image processing unit using a computer or the like, an optical axis 8a of the laser beam is aligned with the center of the respective needle materials 1 by the X-axis drive motor 4, the Y-axis drive motor 5, and the laser beam is then irradiated to open holes. The order of opening holes is needle material 1d, material 1e and material If, for example.
Patent Document 1: Japanese Utility Model Laid-open No. S56-37918
Patent Document 2: Japanese Patent Application Laid-open No. H03-33437

### [Disclosure of Invention]

### [Problem to be Solved by the Invention]

However, the hole opening method of the aforementioned Patent Document 2 has the following problem. Firstly, since the camera 11 photographs the entire collection B of the needle materials 1, the pixel count for photographing the needle materials 1 is lower, and accuracy of identifying the center locations is decreased. Moreover, while the center of the needle materials 1 to be processed by the X-axis drive motor 4 and the Y-axis drive motor 5 are aligned with the optical axis 8a of the laser beam, the needle materials 1 cannot be arranged in a straight line along the X axis or the Y axis, as shown with the needle materials 1d, 1e, and If of FIG. 8. Therefore, when aligning the center of the needle materials 1 to be processed with the optical axis 8a of the laser beam, the X-axis drive motor 4 and the Y-axis drive motor 5 move not only in a single direction but in the reverse direction as well. For example, considering the case of shifting from the needle materials 1d, to 1e, and to If, its moving direction along the Y axis in the case of the needle materials 1d to 1e opposes that in the case of the needle materials 1e to 1f. The same occurring somewhere along the X axis can be easily understood.

While the table 3 can shift precisely in units of several micrometers to several hundred micrometers using ball screws, when shifting in the reverse direction, error equivalent to the backlash occurs. As a result of this error, a problem occurs where the optical axis of the laser beam and the centers of the needle materials 1 are misaligned, and holes are opened at misaligned positions from the centers of the needle materials 1. Particularly, the thinner the needle material 1, the greater adverse influence of this error, and a hole cannot be opened precisely with the needle material 1 having a diameter of 200 micrometers or less by the aforementioned method.

The present invention is devised through consideration of the aforementioned problems. An objective thereof is to provide a hole opening device and a hole opening method for an eyeless needle capable of accurately aligning the center of a needle material and the optical axis of a laser beam as well as opening holes precisely even with a thin needle material of 200 micrometers or less.

### [Means of Solving the Problem]

In order to attain the objective described above, the hole opening device for an eyeless needle, according to the present invention, is characterized by including a table comprising a mounting surface for arranging in parallel a plurality of rod-like needle materials with respective base ends of the plurality of rod-like needle materials aligned in a row; a shifter for shifting the table linearly; a camera for photographing base ends of several needle materials which are a part of the plurality of needle materials arranged in a row; a laser oscillator arranged opposite the base ends for irradiating a laser beam of an optical axis parallel to the needle materials; an image processing means for locating a needle material to be processed from the photographed image taken by the camera and locating the center location of the base end of the located needle material from the image of the base ends of the needle materials; and a control unit for driving the shifter so as to overlap the center location of the base end located by the image processing means and the optical axis of the laser beam irradiated by the laser oscillator.

This device may adopt a configuration further including an adjusting mechanism for adjusting so as for a shifting direction of the table by the shifter to be parallel with a direction in which the needle materials are arranged. Alternatively, the needle material to be processed located by the image processing means is the first needle material in the shifting direction when there is no needle material in which a hole has been opened, while the needle material to be processed located by the image processing means is an adjacent needle material to the last needle material to have a hole opened when there is a needle material in which a hole has been opened. Further alternatively, when shifting the plurality of needle materials, the shifter first shifts the table a distance equivalent to diameter of one needle material, and then shifts a distance equivalent to misalignment between the center of the base end of the needle material to be processed and the optical axis of the laser beam accordingly. Yet even further alternatively, the image processing means determines a needle material to be processed next and locates the center location of that needle material, and the shifter shifts the plurality of needle materials so as to make the center location of the needle material to be processed next and the optical axis of the laser beam match.

In order to attain the objective described above, the hole drilling method for an eyeless needle, according to the present invention, is characterized by including: a step of arranging in parallel a plurality of rod-like needle materials with respective base ends of the plurality of rod-like needle materials aligned in a row, and mounting them on a table; a step of photographing, by a camera, the base ends of several needle materials which are a part of the plurality of needle materials aligned in a row; a step of locating a needle material to be processed from the photographed image and locating the center location of the base end of the located needle material from the image of the base ends of the needle materials; a step of matching the center location of the base end of the needle material to be processed and an optical axis of a laser of a laser oscillator arranged opposite the base ends of the located needle materials for irradiating a laser beam of an optical axis parallel to the needle materials by shifting the table; a step of opening a hole of a predetermined depth along the length of the needle material from the base end by the laser; and a step of locating a needle material to be processed next of the plurality of needle materials from the photographed image taken by the camera and a step of locating a center location of the base end of the located needle material from the image of the base ends of the needle materials, shifting the table so as to match the optical axis of the laser of the laser oscillator and the center location of the needle material to be processed, and opening a hole; wherein these immediately previous two steps are repeated until there are no remaining needle materials.

This method may adopt a structure where after the plurality of rod-like needle materials are mounted on the table, a shifting direction of shifting the table linearly and direction in which the needle materials are arranged are adjusted so as to be parallel. Alternatively, it may further include a step of aligning in parallel and in a row a plurality of rod-like needle materials, fixing them with a tape, and cutting an end of all of the needle materials to form base ends before the step of arranging in parallel the plurality of rod-like needle materials with respective base ends of the plurality of rod-like needle materials aligned in a row, and mounting them on a table. Further alternatively, in the method of locating the needle material to be processed next, the first needle material in the shifting direction is located when there is no needle material in which a hole has been opened while an adjacent needle material to the last needle material in which a hole has been opened is located when there is a needle material in which a hole has been opened. Yet even further alternatively, when shifting the table so as to shift the plurality of needle materials, first shifting a distance equivalent to the diameter of one needle material is carried out, and then shifting a distance equivalent to the misalignment between the center of the base end and the optical axis of the laser beam is carried out. Yet even further alternatively, when shifting the table so as to shift the plurality of needle materials, first, a needle material to be processed next is determined from the image photographed by the camera, a center location of that needle material is located, and the table is shifted so as to match the center of the base end of the needle material to be processed and the optical axis of the laser beam.

The needle materials are aligned on the table, and several of them are photographed by the camera. A needle material to be processed is identified from the camera image, and a center location of that needle material is found from that image. Next, the plurality of needle materials are shifted by shifting the table, the center location of the needle material to be processed and the optical axis of a laser beam are matched, and the laser beam is irradiated so as to open a hole therein.

The table is shifted, a needle material to be processed next is found from the image photographed by the camera, the same process is repeated, and a hole is opened until the last needle material, thereby ending processing.

If a plurality of rod-like needle materials are aligned in parallel, fixed with a tape, and an end of all of the needle materials cut to form base ends, orientation of the base ends may be uniform, allowing easier image analysis.

When opening a hole in the next needle material, adopting a configuration where it is first shifted a distance equivalent to the diameter of one needle material, and then the misalignment between the center of the base end of the processing object needle material and the optical axis of the laser beam is shifted allows shifting quickly. Alternatively, a needle material to be processed next may be determined from the image photographed by the camera and the center location of that needle material found so as to match the center of the base end of the needle material to be processed and the optical axis of the laser beam.

### [Results of Invention]

According to the present invention, since the base ends of several needle materials which are a part of a plurality of needle materials aligned in row are photographed by the camera, the pixel count per needle material may be increased, and center locations may be accurately found. Moreover, according to the present invention, shifting the plurality of needle materials by linearly shifting the table only by the shifter simplifies the configuration. In the case of the present invention, the table shifts only in one direction and not in the reverse direction. Therefore, influence of backlash is omitted and the original highly accurate positioning of ball screws is possible. This allows precise hole opening even in thin needle materials of 200 micrometers or less.

### [Brief Description of Drawings]

[0043]
FIG. 1 is a drawing illustrating the configuration of a hole opening device for an eyeless needle according to the present invention, where 1(a) is a top view and 1(b) is a drawing viewed from A-A;
FIG. 2 is an exemplary photographed image taken by a camera;
FIG. 3 is a block diagram of a hole opening device for an eyeless needle according to the present invention;
FIGS. 4(a) to 4(f) are diagrams describing steps of a process for opening a hole in a needle material;
FIG. 5 is a flowchart for the process of opening a hole;
FIG. 6 is an oblique view of a straight eyeless needle before it is curved;
FIG. 7 is a diagram illustrating the configuration of a conventional hole opening device; and
FIG. 8 illustrates a part of an image of a collection of needle materials photographed by a camera.

### [Description of References]

[0044]
1: needle material
1b: base end
1c: hole
21: table
22a: shifter
25: camera
25a: photographed image
26: laser oscillator
26a: optical axis of laser beam
27a, 27b: dichroic mirror
28: lens
29: tape
30: control unit
31: image processing means
32: input unit
33: display

### [Best Mode for Carrying Out the Invention]

An embodiment according to the present invention is described with reference to accompanying drawings forthwith.

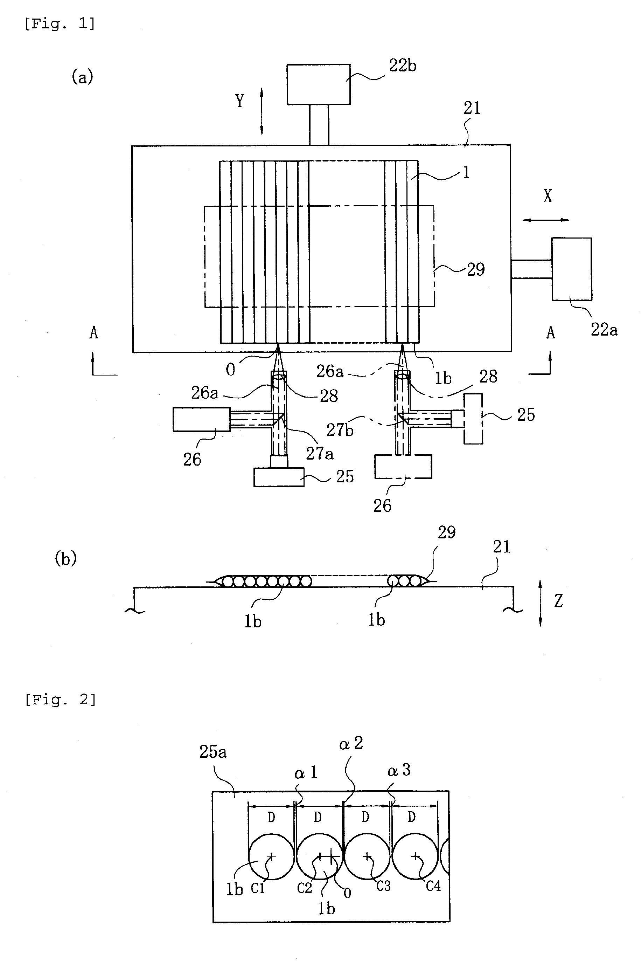

FIG. 1 is a drawing illustrating the configuration of a hole opening device for an eyeless needle according to the present invention, where 1(a) is a top view and 1(b) is a drawing viewed from A-A. The hole opening device of the present invention as shown in these drawings includes a table 21, a shifter 22a, which moves the table 21 in the X-axis direction, a sub-shifter 22b, which moves the table 21 in the Y-axis direction, an auxiliary shifter 22c, which moves the table 21 in the Z-axis direction, and an adjusting device 23, which makes the shifting direction (X axis) of the shifter 22a be parallel to direction in which the plurality of needle materials 1 are deployed. The adjusting device 23 may be controlled manually or by a control unit 30. Ball screws are used in the shifter 22a, the sub-shifter 22b, and the auxiliary shifter 22c, allowing highly accurate positioning. 100 to 200 pieces of the needle material 1, tops and bottoms of which are sandwiched between pieces of a tape 29, are deployed in an orderly line and are fixed on the table 21 by an appropriate means, such as a clamp not illustrated in the drawing. The tape 29 may be an adhesive tape, for example, that is preferably thin and able to temporarily fix the needle material 1. Moreover, in the embodiment illustrated in the drawing, while the tops and bottoms of the needle material 1 are sandwiched between pieces of a tape, it may be provided only just either above or below it. The respective needle materials 1 are arranged in a straight line along the X axis, the base ends 1b are facing downward in FIG. 1(a), and the center locations of the respective needle materials 1 are at a uniform height from the top of the table 21. Afterward, while the needle material 1 illustrated in the drawing has sharpened front ends, this hole opening process may be performed after the front end has been sharpened.

The collection B of the conventional needle materials 1 had inconsistencies in orientation of the base ends of the respective needle materials. Therefore, there is a problem of lack of image sharpness, making analysis difficult. With the present invention, the needle materials 1 may be prepared cut to a predetermined length, and the base ends aligned and fixed by the tape 29, as in the conventional way. However, orientation of the base ends is inconsistent in this case. As a method to resolve this problem, the needle materials 1 are kept longer than the predetermined length, fixed by jigs or the tape 29, and excessive portions are then cut off together. As a result, orientation of the base ends 1b of the respective needle materials 1 are aligned, allowing achievement of a clear image when photographed by a camera and easier image analysis.

A camera 25 is deployed so as to face the base ends 1b on the table 21, and a laser oscillator 26 is deployed perpendicular thereto. Reference numeral 27a denotes a dichroic mirror for reflecting the laser beam and transmitting visible light, and reference numeral 28 denotes a lens. The lens 25, the laser oscillator 26, the dichroic mirror 27a and the lens 28 are the same as those described in the conventional example, and arrangement thereof is also the same.

Virtual lines shown in FIG. 1(a) indicate an example in which the camera 25 and the laser oscillator 26 are used instead. Either those of the solid lines or those of the virtual lines should be used. While the case of the virtual lines also uses a dichroic mirror 27b, the dichroic mirror 27a indicated by a solid line transmits visible light and reflects laser beam, and on the contrary, the dichroic mirror 27b indicated by the virtual line reflects the visible light and transmits the laser beam.
Note that while the camera 25 is a digital camera, a camera for photographing moving pictures may be used.

FIG. 2 is an exemplary photographed image 25a taken by the camera 25. The camera 11 of the conventional example photographed one image of the entire collection B of the needle materials 1. Therefore, the needle materials 1 scatter across the X-Y plane, reducing the pixel count per material. On the contrary, according to the present invention, the camera 25 only photographs several pieces (4 pieces in FIG. 2) of the needle materials 1 aligned in a row. This allows substantial increase in pixel count per needle material 1. This increase in pixel count allows accurate identification of center locations of the needle materials 1.

In FIG. 2, a point O within the photographed image 25a is a fixed point indicating the position of an optical axis 26a of the laser beam. Moreover, the needle materials 1 have the same diameter D and are aligned in a row on the table 21. Points C1 to C4 are not part of the image but indicate center locations of each of the base ends 1b found by the image processing unit described later. While there are cases where the base ends 1b of the respective needle materials 1 are close in contact with each other, most of the time, gaps indicated by α 1 to α 3 are formed.

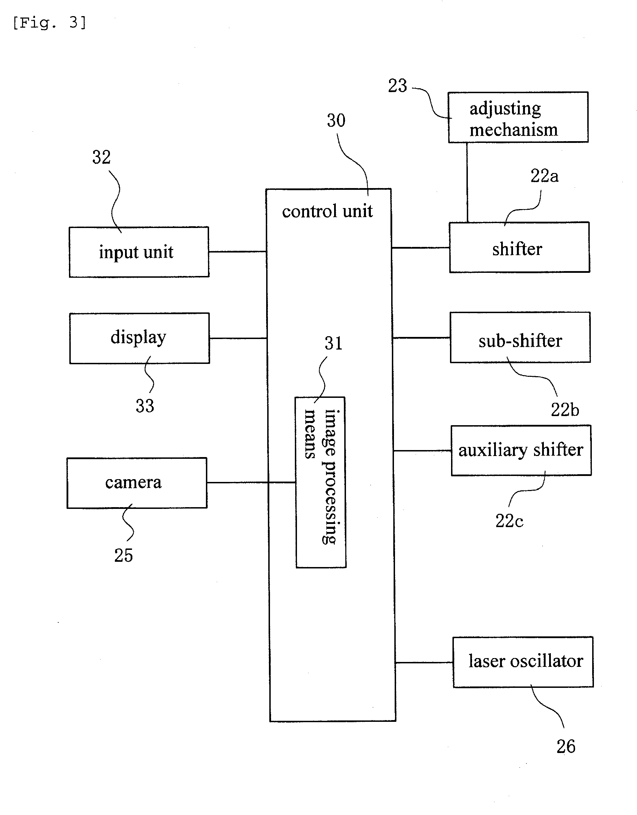

FIG. 3 is a block diagram of a hole opening device for an eyeless needle according to the present invention. The control unit 30 is mainly constituted by a computer, includes a CPU, a flash memory, a storage means, such as a hard disk etc., receives an instruction from an input unit 32 such as a keyboard, operates according to an installed program, and displays various images on a display 33. The control unit 30 includes an image processing means 31, which selects a needle material 1 to be processed from the image, and carries out an operation such as finding the center location of that needle material. The control unit 30 allows shift in the X, Y, and Z axis directions by the shifter 22a (X axis), the sub-shifter 22b (Y axis), and the auxiliary shifter 22c (Z axis), respectively. Since the needle materials 1 are lined up in the X-axis direction, only the shifter 22a (X axis) needs to be instructed in order to overlap the center location of the needle material 1 to be processed and the optical axis 26a of the laser beam. The sub-shifter 22b (Y axis) is used for initial setup such as focusing the laser and the like, and may be operated manually. The auxiliary shifter 22c (Z axis) is only used when adjusting the shifting direction of the shifter 22a (X axis).

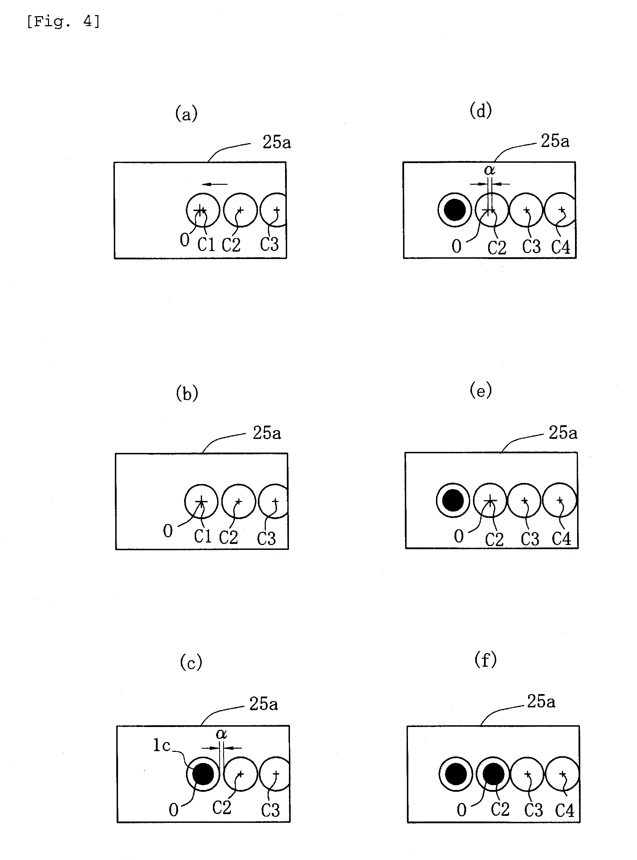

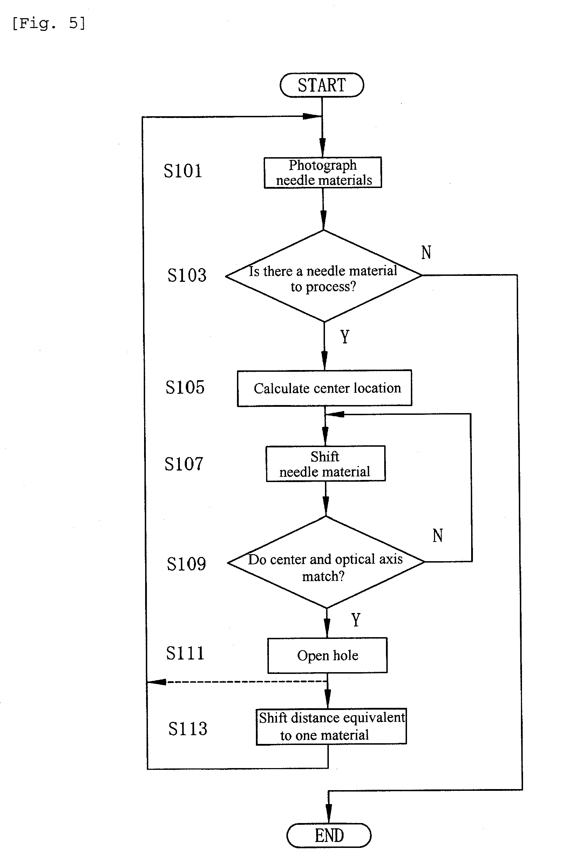

FIGS. 4(a) to 4(f) are diagrams describing steps of a process for opening a hole in the needle material, and FIG. 5 is a flowchart for the process of opening a hole. The method of opening a hole in an eyeless needle according to the present invention will be described using these drawings.

As shown in FIG. 4(a), the camera 25 photographs the first several pieces (3 pieces here) of the plurality of needle materials 1, and displays this image as the photographed image 25a on the display 33 (step S101). The image processing means 31 detects the plurality of needle materials 1 by grasping the circular outlines of the respective base ends of the plurality of needle materials 1 in the photographed image 25a.

Since a hole has not been opened in any of the needle materials 1, the first needle material 1 in the feeding direction (the far left needle material 1 in FIG. 4(a)) is determined to be processed (step S103). Once the needle material 1 to be processed is determined, center C1 of the base end 1b is located by finding the circular center location forming the base end 1b of that needle material 1 (step S105). Center points C2 and C3 may be found for the second and third needle materials 1 but is not always necessary.

In FIG. 4(a), there is a misalignment between the center C1 of the first needle material and point O or optical axis of the laser beam. The image processing means 31 then finds this misalignment through calculation. The control unit 30 calculates the actual shifting distance of the table 21 from the distance on the photographed image 25a, and then sends an instruction to the shifter 23. The shifter 23 shifts the table 21 only the provided distance and then stops (step S107). The camera 25 photographs the state after shifting, and the image processing means 31 checks that the center C1 and the point O are overlapped as shown in FIG. 4(b) (step S109). If they are not overlapped, the above operation is repeated. Moreover, depending on size, allowable difference of the misalignment between the point O or optical axis of the laser beam and the center C1 of the needle material may be set within a range of ±5 pixels, for example.

According to the present invention, the needle materials 1 are aligned in a row on the table 21. Therefore, in order to overlap the center C1 and the point O, the table 21 needs to be shifted only in the X-axis direction by the shifter 22a, where the sub-shifter 22b and the auxiliary shifter 22c are normally not used.

Moreover, in FIG. 4(a), it is important that the center C1 is misaligned posteriorly in the feeding direction of the point O. In order to avoid backlash influence of the shifter 22, the table 21 is always shifted in one direction (arrow direction shown in FIG. 4(a)) so as to overlap the center C1 and the point O. If the center C1 is misaligned anteriorly in the feeding direction of the point O in FIG. 4(a), shift back the table 21 by the shifter 22 to more than the misalignment so that the center C1 is posteriorly in the feeding direction of the point O before shifting it in the arrow direction so as to overlap them. However, this operation is unnecessary for the second and further subsequent needle materials, and only the shifter 22 shifting the table 21 in the feeding direction is required.

Once the center C1 and the point O are confirmed to be overlapping as shown in FIG. 4(b), a predetermined output pulse is irradiated from the laser oscillator 26 for a predetermined time. This opens the hole 1c in the first needle material 1 as shown in FIG. 4(c) (step S111).

Next, the shifter 22 shifts the table 21 only a distance equivalent to the diameter D of the needle material 1 (step S113). Returning to step S101, the needle material 1 is then photographed. FIG. 4(d) illustrates the photographed image 25a after it has been shifted. Since the hole 1c is opened in the first needle material 1 in this photographed image 25a, the second needle material 1 is recognized as the one to be processed (step S103). The center C2 of the base end 1b of the second needle material 1 is then found (step S105). As shown in FIG. 4(c), since there is a gap α between the first needle material 1 and the second needle material 1, a misalignment α results between the center C2 and point O of the second material. This distance is found by the image processing means 31, an instruction is sent to the shifter 22 from the control unit 30, and the table 21 is shifted only α (step S107). The shifting direction in this case is the same direction in which the previous material was shifted only a distance equivalent to the diameter D. Accordingly, the problem of backlash does not occur. Moreover, since the step of shifting the table 21 only a distance equivalent to the diameter D of the needle material 1 in step S113 makes it move only a determined distance, it can be performed quickly, and allows reduction in positioning time on the whole.

Once the center C2 of the second needle material 1 and the point O are confirmed to be matching as in FIG. 4(e) (step S109), a laser beam is irradiated to open the hole 1c in the second needle material 1 as shown in FIG. 4(f) (step S111). Afterward, it shifts only a distance equivalent to the diameter D of the needle material 1, returns to step S101 and repeatedly opens holes until there are no remaining needle materials 1, ending the process.

Aside from the above shifting method, the method described next may be employed as well. Once hole-opening is completed in step S111, processing returns to step S101 as indicated by a dotted line, and the next image is photographed. A needle material to be processed next is determined (step S103) and the center location thereof is found (step S105). The new center location is located in the image and that center is shifted to the center point of the laser (step S107). The match is confirmed in step S109 and a hole is opened. In this case, shifting of the table only requires the shifting of step S107 and the shifting of step S113 may be omitted.

When the image processing means 31 is locating the center in the aforementioned process, even if there is an impairment for some reason in a part of the circular base end, or spatter adheres thereto at the time of opening a hole, thereby a part of the circle not being displayed in the image, the center location can be found from the remaining circular portion. Aside from the above, an image processing method such as a pattern matching method using normalized correlation may be used.

While the cross-sectional shape of the needle material 1 is circular in the above embodiment, the same processes may be performed in the case of shapes other than a circle such as a triangle or a trapezoid.

## Claims

1. A hole opening device for an eyeless needle, comprising: a table comprising a mounting surface for arranging in parallel a plurality of rod-like needle materials with respective base ends of the plurality of rod-like needle materials aligned in a row; a shifter for shifting the table linearly; a camera for photographing base ends of several needle materials which are a part of the plurality of needle materials aligned in a row; a laser oscillator arranged opposite the base ends for irradiating a laser beam of an optical axis parallel to the needle materials; an image processing means for locating a needle material to be processed from the photographed image taken by the camera and locating the center location of the base end of the located needle material from the image of the base ends of the needle materials; and a control unit for driving the shifter so as to overlap the center location of the base end located by the image processing means and the optical axis of the laser beam irradiated by the laser oscillator.

2. The hole opening device for an eyeless needle according to Claim 1, further comprising an adjusting mechanism for adjusting so as for a shifting direction of the table by the shifter to be parallel with a direction in which the needle materials are arranged.

3. The hole opening device for an eyeless needle according to Claim 1 or Claim 2, wherein the needle material to be processed located by the image processing means is the first needle material in the shifting direction when there is no needle material in which a hole has been opened, while the needle material to be processed located by the image processing means is an adjacent needle material to the last needle material to have a hole opened when there is a needle material in which a hole has been opened.

4. The hole opening device for an eyeless needle according to any one of Claims 1 to 3, wherein when shifting the plurality of needle materials, the shifter first shifts the table a distance equivalent to diameter of one needle material, and then shifts a distance equivalent to misalignment between the center of the base end of the needle material to be processed and the optical axis of the laser beam accordingly.

5. The hole opening device for an eyeless needle according to any one of Claims 1 to 3, wherein the image processing means determines a needle material to be processed next and locates the center location of that needle material, and the shifter shifts the plurality of needle materials so as to make the center location of the needle material to be processed next and the optical axis of the laser beam match.

6. A hole opening method for an eyeless needle, comprising: a step of arranging in parallel a plurality of rod-like needle materials with respective base ends of the plurality of rod-like needle materials aligned in a row, and mounting them on a table; a step of photographing, by a camera, the base ends of several needle materials which are a part of the plurality of needle materials aligned in a row; a step of locating a needle material to be processed from the photographed image and locating the center location of the base end of the located needle material from the image of the base ends of the needle materials; a step of matching the center location of the base end of the needle material to be processed and an optical axis of a laser of a laser oscillator arranged opposite the base ends of the located needle materials for irradiating a laser beam of an optical axis parallel to the needle materials by shifting the table; a step of opening a hole of a predetermined depth along the length of the needle material from the base end by the laser; and a step of locating a needle material to be processed next of the plurality of needle materials from the photographed image taken by the camera and a step of locating a center location of the base end of the located needle material from the image of the base ends of the needle materials, shifting the table so as to match the optical axis of the laser of the laser oscillator and the center location of the needle material to be processed, and opening a hole; wherein these immediately previous two steps are repeated until there are no remaining needle materials.

7. The hole opening method for an eyeless needle according to Claim 6, wherein after the plurality of rod-like needle materials are mounted on the table, a shifting direction of shifting the table linearly and direction in which the needle materials are arranged are adjusted so as to be parallel.

8. The hole opening method for an eyeless needle according to Claim 6 or Claim 7, further comprising a step of aligning in parallel and in a row a plurality of rod-like needle materials, fixing them with a tape, and cutting an end of all of the needle materials to form base ends before the step of arranging in parallel the plurality of rod-like needle materials with respective base ends of the plurality of rod-like needle materials aligned in a row, and mounting them on a table.

9. The hole opening method for an eyeless needle according to any one of Claims 6 to 8, wherein in the step of locating the needle material to be processed next, the first needle material in the shifting direction is located when there is no needle material in which a hole has been opened while an adjacent needle material to the last needle material in which a hole has been opened is located when there is a needle material in which a hole has been opened.

10. The hole opening method for an eyeless needle according to any one of Claims 6 to 9, wherein when shifting the table so as to shift the plurality of needle materials, first shifting a distance equivalent to the diameter of one needle material is carried out, and then shifting a distance equivalent to the misalignment between the center of the base end and the optical axis of the laser beam is carried out.

11. The hole opening method for an eyeless needle according to any one of Claims 6 to 9, wherein when shifting the table so as to shift the plurality of needle materials, first, a needle material to be processed next is determined from the image photographed by the camera, a center location of that needle material is located, and the table is shifted so as to match the center of the base end of the needle material to be processed and the optical axis of the laser beam.
